# EUROPEAN PATENT APPLICATION

(11) **EP 3 919 589 A1**
(43) Date of publication of application: **08.12.2021**
(21) Application number: 20749760.3
(22) Date of filing: 22.01.2020
(51) Int. Cl.: C10G 11/00

(54) **METHOD FOR CATALYTIC CONVERSION OF HYDROCARBON WITH DOWNER REACTOR AND DEVICE THEREOF**

(30) Priority: 30.01.2019 CN 201910093270
(71) Applicant: Li, Qunzhu, Luoyang, Henan 471003 (CN)
(72) Inventor: LI, Li, Luoyang, Henan 471003 (CN); LI, Qunzhu, Luoyang, Henan 471003 (CN)
(74) Representative: Laine IP Oy
(86) International application number: PCT/CN2020/073675
(87) International publication number: WO 2020/156398

(57) **Abstract**

Provided are a method for the catalytic conversion of hydrocarbons with a downer reactor and a device thereof. The specific process of the method is as follows: a raw material of hydrocarbons after being pre-heated (or not) and a low-temperature regenerant from a regenerant cooler entering an entry end of a downer reactor, flowing down along the reactor for reactions such as catalytic cracking, and a mixture of a reactive oil and gas and a catalyst descending to the end of the reactor for rapid separation, thereby achieving the rapid separation of the catalyst and the oil and gas. The main operation conditions thereof are as follows: the reaction temperature is 460 to 680°C, the reaction pressure is 0.11 to 0.4 MPa, the contact time is 0.05 to 2 seconds, and the weight ratio of the catalyst to the raw material (a catalyst-to-oil ratio) is 6 to 50. The separated catalyst to be regenerated (abbreviated as a spent agent) is stripped by means of a stripper, and enters a regenerator and is burned for regeneration, wherein the regeneration temperature is controlled at 630-730°C. The regenerant from the regenerator enters the regenerant cooler to be cooled to 200-720°C, and then enters the downer reactor for recycling.

## Description

### Cross-reference to Related Applications

This application claims the priority to Chinese patent application CN 201910093270.4, entitled "Method for catalytic conversion of hydrocarbon with downer reactor and device thereof' and filed on January 30, 2019, the entirety of which is incorporated herein by reference.

### Field of the Invention

The present invention belongs to methods for catalytic conversion of hydrocarbons, and in particular relates to catalytic conversion of hydrocarbons using a downer reactor.

### Background of the Invention

Due to the continuous increase in crude oil prices, the growing demand for light oil, and increasingly strict environmental regulations, the use of catalytic cracking technology to process heavy feedstock oil, the production of cleaner fuel products, and the reducing of emission from catalytic cracking units themselves have become hot spots in technology development.

During a catalytic cracking reaction in a riser, the preheated raw material is contacted and vaporized with a high-temperature regenerated catalyst from the regenerator, and then goes into a reaction for a reaction time of about 2-3 seconds. Studies have shown that the activity of the catalyst at the outlet of the riser is only about 1/3 of the initial activity thereof, and when the reaction is carried out for about 1 second, the activity of the catalyst is reduced by about 50%. During the reaction in the riser, the coke formed is deposited on the surface and the active centers of the catalyst, drastically reducing the activity of the catalyst, greatly weakening the catalytic effect, and thus increasing thermal cracking reactions and producing more dry gas and coke.

Riser reactors and downer reactors both have their own advantages. A riser reactor has the advantages of high catalyst concentration, large gas-solid contact area, and high gas-solid contact efficiency. However, since the gas and the solid in the riser flow co-currently against the gravity field, the axial and radial flows in the riser are not uniform, and the catalyst slips down and backmixes greatly and the residence time distribution thereof is not uniform. In contrast, in a downer reactor, since the gas and the solid flow co-currently following the gravity field, the radial flow is more uniform, and the catalyst does not back-mix axially, and the radial distribution of particle concentration and velocity is significantly improved compared with those in a up-flow riser. The downer reactor is thus especially suitable for catalytic conversion reactions characterized by ultra-short contact (reaction) time (typically 1-3 times shorter than that for a riser) and ultra-large catalyst-to-oil ratio (typically 1-3 times larger than that for a riser), such as deep catalytic conversion of residual oil, catalytic thermal pyrolysis, catalytic cracking of gasoline to olefins, etc., and can fully utilize the initial activity of the catalyst, improve the yield of light oil, and reduce the formation of dry gas and coke.

Therefore, domestic and overseas research is generally focused on the development of the inlet structure of downer reactors, gas-solid rapid separation, coupling of riser reactors, and the development of high-activity catalysts, in order to strengthen the mixing of the raw material with the catalyst at the inlet, and improve the total conversion rate and reaction selectivity. CN 1113689C discloses a gas-solid co-current flowing folding-type fast fluidized bed reactor. CN 1162514 C discloses a gas-solid co-current down-flow and up-flow coupled catalytic cracking reactor.

Studies have shown that increasing the concentration of the catalyst in the downer reactor can improve the conversion rate and reaction selectivity of the catalytic conversion reaction in the downer reactor to obtain a relatively high yield of target products. However, there is no report on results of research and development of technical solutions or measures about how to improve the catalyst-to-oil ratio.

In fact, in order to improve coke burning efficiency and regeneration effects, it is usually necessary to use a high regeneration temperature (such as 700-730°C), and therefore temperatures of regenerated catalysts are all very high, much higher than a temperature of a regenerated catalyst required by a downer reactor system for thermal equilibrium. In other words, in order to significantly increase the catalyst-to-oil ratio, it is a must to lower the temperature of the regenerated catalyst, only through which the thermal equilibrium of the reaction system may be maintained. Meanwhile, the ultra-short reaction time also has to be ensured by providing an ultra-large catalyst-to-oil ratio. Otherwise, trying to realize a high conversion rate in an ultra-short reaction time by increasing the reaction temperature will inevitably intensify undesired reactions such as thermal cracking, leading to the increase of the yield of coke and dry gas, and the decrease of the yield of target products such as gasoline and light olefins.

An objective of the present invention is to use, on the condition of ensuring a good regeneration effect, a cold regenerated catalyst circulation method to achieve a large catalyst-to-oil ratio, reduce the temperature of the regenerated catalyst entering the downer reactor, and break the thermal equilibrium limitation of the downer reactor system, to thereby increase the circulation amount of the catalyst, increase the concentration of the catalyst in the downer reactor, increase the activity and the number of active centers of the catalyst, promote desired reactions such as catalytic conversion, hydrogen transfer, isomerization, aromatization, etc., decrease undesired reactions such as thermal cracking, etc., and improve the reaction selectivity, to thus increase the yield of target products such as gasoline and light olefins, and reduce the yield of coke and gases.

Another objective of the present invention is to optimize the temperature of the reaction in the downer reactor by adjusting the temperature of the regenerated catalyst entering the downer reactor, to optimize the temperature distribution in the downer reactor and to optimize the reaction depth and products distribution by means of a suitable reaction temperature and a suitable catalyst-to-oil ratio, to thereby further improve reaction selectivity, reduce the yield of coke and gases, and improve the yield of targets products such as gasoline and light olefins, etc..

### Summary of the Invention

The technical problems to be solved by the present invention are: to use a regenerated catalyst cooling technology to break the thermal equilibrium limitation of the downer reactor system, so as to truly realize a large catalyst-to-oil ratio operation and improve the concentration and the activity of the catalyst in the downer reactor; and also to use a suitable reaction temperature (relatively lower 0-50°C ) to optimize the temperature distribution in the downer reactor to achieve optimal control of the reaction depth, so as to improve the reaction selectivity, increase the yield of light olefins such as propylene etc. and gasoline, increase the aromatic content in gasoline, reduce olefins content in gasoline, and meanwhile reduce the yield of coke and dry gas.

The present invention provides a method for catalytic conversion of a hydrocarbon using a downer reactor. A regenerated catalyst from a regenerator, after being cooled by a regenerated catalyst cooler, enters a downer reactor and is mixed and contacted with a hydrocarbon raw material at an inlet end of the downer reactor; the regenerated catalyst and the hydrocarbon raw material go into a catalytic conversion reaction of the hydrocarbon in the downer reactor, and flow co-currently downward to a tail end of the downer reactor for rapid separation. A separated spent catalyst, after being stripped, enters a regenerator and is burned for regeneration, and a regenerated catalyst, after being cooled by a regenerated catalyst cooler, is returned and recycled to the downer reactor for reuse. A specific process of the method is as follows.
1) The hydrocarbon raw material, after being preheated (or being not preheated), and the low-temperature regenerated catalyst from the regenerated catalyst cooler enter the inlet end of the downer reactor, and react while flowing downward along the reactor. When a mixture of the reacting oil and gas and the catalyst flows downward to the tail end of the reactor, a rapid separation is performed to realize rapid separation of the catalyst and the oil and gas. The downer reactor is operated under the following main conditions: a reaction temperature of 460-680°C (preferably 480-660°C, most preferably 490-650°C), a reaction pressure of 0.11-0.4 MPa, a contact time of 0.05-2 seconds (preferably 0.1-1.5 seconds), and a weight ratio of the catalyst to the raw material (catalyst-to-oil ratio) of 6-50 (preferably 8-40).
2) The separated spent catalyst, after being stripped by a spent catalyst stripper, enters the regenerator and is burned for regeneration. The regeneration temperature is controlled at 630-800°C (preferably 630-730°C, most preferably 650-730°C).
3) The regenerated catalyst from the regenerator enters the regenerated catalyst cooler and is cooled to 200-720°C, and the cooled regenerated catalyst (referred to as cold regenerated catalyst) is recycled to the inlet end of the downer reactor for reuse; or a hot regenerated catalyst (i.e., the regenerated catalyst from the regenerator that is not cooled) bypass is arranged, so that a portion of the hot regenerated catalyst is mixed with the cold regenerated catalyst and then the mixed regenerated catalyst is recycled to the inlet end of the downer reactor for reuse.

Further, the reaction temperature of the downer reactor is controlled mainly by adjusting the catalyst-to-oil ratio (i.e., by providing a control element such as a slide valve, a plug valve, and the like, on a pipe conveying the cold regenerated catalyst), or/and by adjusting the temperature of the cold regenerated catalyst or the temperature of the mixed regenerated catalyst, to maintain the reaction temperature at an optimum value.

By adjusting the temperature of the regenerated catalyst entering the downer reactor, the purpose of adjusting the reaction temperature of the downer reactor is achieved; and further by adopting a suitable reaction temperature and a suitable catalyst-to-oil ratio, the temperature distribution of the downer reactor is optimized, and the reaction depth and product distribution are optimized, which further improves the reaction selectivity, reduces the yield of coke and offgas., and improves the yield of target products such as gasoline and light olefins etc.

The cold regenerated catalyst temperature is controlled by adjusting the flow rate of a fluidizing medium entering the regenerated catalyst cooler and/or the flow rate of the cold catalyst returned to the regenerator and/or other parameters. The fluidizing medium may be air, a steam, or other gases, or a mixture thereof. The heat-removing medium may be water, a steam, air or other gases, various oils, or the like, or a mixture thereof.

The temperature of the mixed regenerated catalyst entering the downer reactor may be independently controlled by adjusting proportions of the cold regenerated catalyst and the hot regenerated catalyst.

The temperature of the cold regenerated catalyst entering the downer reactor is controlled mainly by adjusting the flow rate of the fluidizing medium and/or the flow rate of the heat-removing medium or/and other parameters; or the temperature of the cold regenerated catalyst is controlled mainly by adjusting the flow rate the fluidizing medium and/or of the heat-removing medium and/or the flow rate of the cold catalyst returned to the regenerator or/and other parameters. Thus, both the catalyst-to-oil ratio (the weight ratio of the regenerated catalyst to feed) and the reaction temperature of the downer reactor can be independently controlled.

There are of course many other control devices and control methods, and the implementation of the inventive concept of the present invention is not limited in this respect.

According to the method and the device of the present invention, it is also possible to provide a catalyst mixing and buffering space downstream of the regenerated catalyst cooler to enhance mixing of the regenerated catalyst and eliminate radial temperature differences caused by non-uniform heat-removing and non-uniform flows (so as to get the temperature of the regenerated catalyst uniform)) so as to meet the requirements of downstream reaction temperature control, and improve the accuracy and flexibility of downstream reaction temperature control. At the same time, this can also improve the density of the regenerated catalyst, and increase the driving force of the regenerated catalyst circulation system to overcome the increase in the resistance of the circulation system caused by the increase in the catalyst-to-oil ratio, thereby realizing a large catalyst-to-oil ratio operation.

The catalyst mixing and buffering space is operated by a low-velocity dense-phase fluidized bed having a superficial gas velocity (the ratio of the flow rate of the fluidized medium to the empty cross-section of the device) of less than 0.3 m/s (preferably 0.0001-0.1999 m/s).

The fluidizing medium for the catalyst mixing and buffering space may be air, a steam, or other gases, or a mixture thereof (preferably a steam) used to reduce the amount of air entrained by the circulating catalyst, reduce the content of non-hydrocarbon gases such as nitrogen etc. in the dry gas, improve the calorific value of the dry gas, and reduce the power consumption of a rich gas compressor. Its specific structure, connections, operations, and control processes are well-known to those skilled in the art, and the implementation of the inventive concept of the present invention is not limited in this respect.

The regenerated catalyst cooler connected each other to the reactor described above may be arranged outside the regenerator or inside the regenerator. The regenerated catalyst cooler may be integrated with the regenerator, the downer reactor and/or the catalyst mixing and buffering space, or may be connected thereto through conveying pipes. The specific structure, connections, operations, and control processes of the regenerated catalyst cooler are well-known to those skilled in the art, and the implementation of the inventive concept of the present invention is not limited in this respect.

The catalyst cooler is a mature industrial device, for which the method and the device of the present invention can adopt various types of structures (such as an up-flow type, a down-flow type, etc.). Various specific connection structures (such as an inner circulation pipe, a Y-type or U-type external conveying (circulation) pipe, etc.) may also be adopted for the catalyst conveying channel, with or without a degassing (balancing) pipe. Its specific structure, connections, operations and control processes are well-known to those skilled in the art, and the implementation of the inventive concept of the present invention is not limited in this respect.

The hydrocarbon raw material of the present invention may be any heavy oil having been hydrogenated or having not been hydrogenated, including one of or a mixture of two or more than two of straight-run gas oil (distillate oil)(VGO), coking gas oil (distillate oil)(CGO), hydrocracked tail oil(HVGO), atmospheric pressure residual oil, vacuum residual oil, shale oil, synthetic oil, crude oil, coal tar, recycle oil, oil slurry, deasphalted oil, thermal cracking heavy oil, viscosity-reduced heavy oil, heavy diesel, and the like. The gas oil (distillate oil) fraction includes high-density cycloalkyl or naphthenic intermediate gas oil (distillate oil). The gas oil (distillate oil) fraction may be a full-range fraction, such as a fraction in a range of from an initial boiling point to about 565°C, or may be a partial narrow fraction thereof, such as a fraction in a range of 450-520°C.

The hydrocarbon raw material of the present invention may also be a light hydrocarbon raw material, which is an olefin-containing hydrocarbon or saturated liquid light hydrocarbon in a refinery or a petrochemical plant, including one of liquefied petroleum gas, and light oil, or a mixture of more than one thereof in any ratio. The liquid light hydrocarbon may be C4 and C5 fractions containing butene and pentene, or a mixture thereof in any ratio. The light oil may be a gasoline fraction, including one or two or more of straight-run gasoline, gas condensate, catalytic cracking gasoline, thermal cracking gasoline, viscosity-reduced gasoline, coking gasoline, pyrolysis gasoline, or a mixture gasoline thereof in any ratio, and may be a full-range gasoline such as a fraction in a range of from an initial boiling point to about 220°C, or a partial narrow fraction thereof such as a fraction in a range of from an initial boiling point to 80°C. The light oil may also be a diesel fraction, including catalytic cracking diesel, and may be a full-range diesel such as a fraction in a range of an initial boiling point to about 365°C, or a partial fraction thereof such as a fraction in a range of from an initial boiling point to 300°C.

The method for catalytic conversion according to the present invention may be implemented separately or used in combination with a riser reactor. For example, it is possible to use the gas-solid co-current flowing folding-type fast fluidized bed reactor disclosed in CN 1113689C or the gas-solid co-current down-flow and up-flow coupled catalytic cracking reactor disclosed in CN 1162514C.

The method and the device of the present invention may adopt various reaction regeneration modes, such as providing a first regenerator, a second regenerator, etc. Its combinations, operations and control processes are well-known to those skilled in the art, and the implementation of the inventive concept of the present invention is not limited in this respect.

When using the method and the device of the present invention, both the separation of the reaction products and the regeneration of the catalyst are carried out according to conventional methods. The spent catalyst is burned for regeneration in the regenerator under a conventional regeneration condition of a catalyst used for catalytic conversion, and the regeneration temperature is usually controlled at 650-800°C (preferably 630-730°C, most preferably 650-730°C).

The method and the device of the present invention may adopt any commercially used catalytic conversion catalysts and auxiliaries, including ZSM catalysts maximizing propylene production, ultra-stable molecular sieve catalysts, and the like.

The fluid catalytic conversion process and device are mature industrial process, and its combinations, operations, control processes, as well as operation conditions (such as feed temperature, reaction temperature, reaction pressure, contact time, and catalyst-to-oil ratio, etc.) and selection for catalysts are well-known to those skilled in the art, and the implementation of the inventive concept of the present invention is not limited in this respect.

In order to ensure a proper temperature for burning the catalyst for regeneration and maintain the thermal equilibrium of the reaction regeneration system, one or two or more of the following measures may be taken separately or in combination.
1) Any one or two or more of a combustible solid, a liquid fuel, and a gaseous fuel, or a mixture thereof may be injected into the regenerator.
2) The main wind (air used for burning) entering the regenerator may be used to exchange heat with the regenerated flue gas, or may be used as a heat-removing medium for the regenerated catalyst cooler to exchange heat with the regenerated catalyst, so that the temperature of the main wind is increased by 160-650°C (preferably 200-520°C) before the main wind enters the regenerator.
3) The main wind, before entering the regenerator, may be heated to 200-1800°C (preferably 600-1500°C) with one or two or more of or a mixture of a solid fuel, a liquid fuel, and a gaseous fuel.

Compared with the existing technologies, the present invention has the following advantages.

The method for catalytic conversion according to the present invention employs a cold regenerated catalyst circulation technology that breaks the thermal equilibrium in the reactor and the thermal equilibrium in the reaction regeneration system, making it possible to achieve a large catalyst-to-oil ratio and optimize the operating conditions of the downer reactor (for example, an ultra-short reaction time and a larger catalyst-to-oil ratio may be used), so as to achieve optimal control of the reaction depth at a suitable (relatively low) reaction temperature, greatly promote desired reactions such as catalytic conversion (cracking), and inhibit undesired reactions such as thermal cracking, thereby increasing the reaction selectivity.
1. The present invention increases the circulation amount of the catalyst, decreases the coking rate of the catalyst (i.e., the carbon difference between the regenerated catalyst and the spent catalyst), and increases the number of active centers per the catalyst in the downer reactor.
2. The present invention increases the circulation amount of the catalyst, improves the concentration of the catalyst in the downer reactor, increases the contact area between the oil and the catalyst, improves the contact effect between the oil and the catalyst, and meanwhile increases the number of active centers of the catalyst contacted with the per unit raw material, thus greatly promoting desired reactions such as catalytic cracking, hydrogen transfer, isomerization, aromatization, etc.
3. The present invention increases the circulation amount of the catalyst, significantly reduces the temperature difference between the inlet and outlet in the downer reactor, optimizes the temperature distribution of the downer reactor, and thus effectively inhibits undesired reactions such as thermal cracking.
4. The present invention adopts circulation of a low-temperature regenerated catalyst, which can help to reduce hydrothermal deactivation of the regenerated catalyst during conveying of the regenerated catalyst (before entering the downer reactor), increase the activity of the regenerated catalyst, and reduce the consumption of the catalyst.
5. The present invention adopts circulation of a low-temperature regenerated catalyst, which can help to improve the feed temperature, improve the atomization effect of the feedstock oil, and together with the high-activity catalyst, the large catalyst-to-oil ratio and the suitable reaction temperature, produces a synergistic effect in increasing the yield of the target products such as gasoline and light olefins etc. by 2.0-3.0 percentage points and the octane number of gasoline by 0.5-2.0 units while greatly reducing the yield of undesired products (such as coke and offgas etc), by way of which the economic efficiency of the technology is improved.
6. The providing of the catalyst mixing and buffering space downstream enhances the mixing of the catalyst, and enables the temperature of the regenerated catalyst to reach an equilibrium and to be uniform and stable, thereby improving the accuracy and flexibility of downstream reaction temperature control.
   At the same time, the providing of the catalyst mixing and buffering space also effectively increases the density and the buffer capacity of the regenerated catalyst, and improves the driving force of the regenerated catalyst circulation system, thereby improving the safety, reliability, stability, controllability, and flexibility of operations, and realizing optimal control of the reaction temperature and reaction depth.
7. Use of a steam as the fluidized medium helps to eliminate the air entrained by the circulating catalyst and eliminate non-hydrocarbon gases such as nitrogen etc. in the dry gas, thereby improving the calorific value of the dry gas, reducing the power consumption of the rich gas compressor, and reducing the size and consumption of devices in the gas separation section.
8. In the method for catalytic conversion using the downer reactor according to the present invention, adjustment of reaction conditions such as the reaction temperature and the catalyst-to-oil ratio can be realized relatively independently, which allows more flexibility and allows flexible adjustment depending on the type of the raw material and market conditions to achieve different product distribution. Examples are as follows.

1) A relatively low reaction temperature may be used to produce a low-olefin high-octane gasoline.
   In this case, a relatively low reaction temperature (for example, 460-520°C, preferably 490-510°C) may be used to promote desired reactions such as catalytic cracking, isomerization, aromatization, etc. to produce a low-olefin high-octane gasoline.
2) A relatively high reaction temperature may be used to maximally produce light olefins such as ethylene and propylene etc., and to produce chemical raw materials such as aromatic hydrocarbons etc..

When it is necessary to maximally produce light olefins and aromatic hydrocarbons, a very high reaction temperature (for example, 520-650°C, preferably 540-630°C) may be used, so that reactions such as olefin cracking and aromatization etc. dominate, thereby maximally producing light olefins such as ethylene and propylene while producing a gasoline blending component with a high aromatic content, in which case chemical raw materials such as aromatic hydrocarbons may be produced by extraction of aromatic hydrocarbons.

### Brief Description of the Drawings

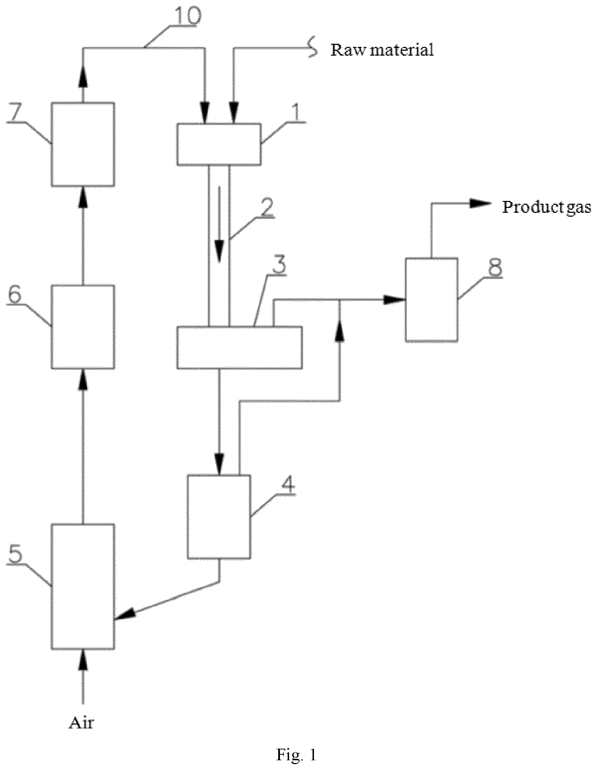

Figs. 1-3 are typical schematic diagrams of devices for catalytic conversion using a downer reactor according to the present invention.

The present invention is described in detail below in connection with the accompanying drawings. The drawings are drawn to illustrate the present invention and are not intended to limit any particular implementation of the inventive concept of the present invention.

Fig. 1 is a block flow diagram of a method for catalytic conversion using a downer reactor according to the present invention.

As shown in Fig. 1, the method for catalytic conversion of the present invention comprises an inlet end 1 of the downer reactor, the downer reactor 2, a rapid separation unit 3, a spent catalyst stripper 4, a regenerator 5, a regenerated catalyst cooler 6, a catalyst mixing and buffering space 7, and a secondary separator 8.

A feedstock oil, after being preheated and warmed up, enters a feedstock nozzle. The feedstock oil is atomized under the action of an atomizing steam, at which time the feedstock oil enters, in the form of fine droplets, a mixing section in the downer reactor 2. In the meantime, a high-temperature catalyst coming from the regenerator 5, after being cooled by the regenerated catalyst cooler 6 and then passed through the catalyst mixing and buffering space 7 located at the downstream location (or at a lower portion) to reach temperature equilibrium, enters the inlet end 1 of the downer reactor and then enters the downer reactor 2 and is mixed with the atomized feedstock oil. The gas phase and the solid phase both are rapidly contacted and thoroughly mixed with each other in the mixing section, then flow co-currently downward along the reactor 2 while undergoing a catalytic conversion reaction. When reaction products and the catalyst flow, in a mixed manner, co-currently downward to a tail end of the downer reactor 2, the gas-solid rapid separation unit 3 rapidly separates the catalyst and the product oil and gas, or the product oil and gas is quenched (to avoid a secondary reaction; not shown in the figure) and enters the secondary separator 8 (such as a cyclone separator) for further removal of the catalyst and then enters a downstream fractionation or separation system for further separation to obtain desired gas products and liquid products. The main operating conditions are as follows: a reaction temperature of 460-680°C (preferably 480-660°C, most preferably 490-630°C); a reaction pressure of 0.1-0.4 MPa; a contact time of 0.05-2 seconds (preferably 0.1-1.5 seconds); and a weight ratio of the catalyst to the feedstock oil (catalyst-to-oil ratio) of 6-50 (preferably 8-40).

The separated spent catalyst is stripped by the spent catalyst stripper 4 and then enters the regenerator 5 and is burned for regeneration. The regeneration temperature is controlled at 630-730°C (preferably 650-730°C).

The regenerated catalyst from the regenerator 5 enters the regenerated catalyst cooler 6 and is cooled to 200-720°C and then directly returned and recycled to the downer reactor 2 for reuse; or the cold regenerated catalyst leaving a lower portion or a bottom of the regenerated catalyst cooler 6, after being passed through the mixing buffering space 7 for mixing and buffering to reach temperature equilibrium, is returned and recycled to the downer reactor 2 for reuse. The fluidizing medium used may be air, a steam, other gases, or a mixture thereof (preferably a steam).

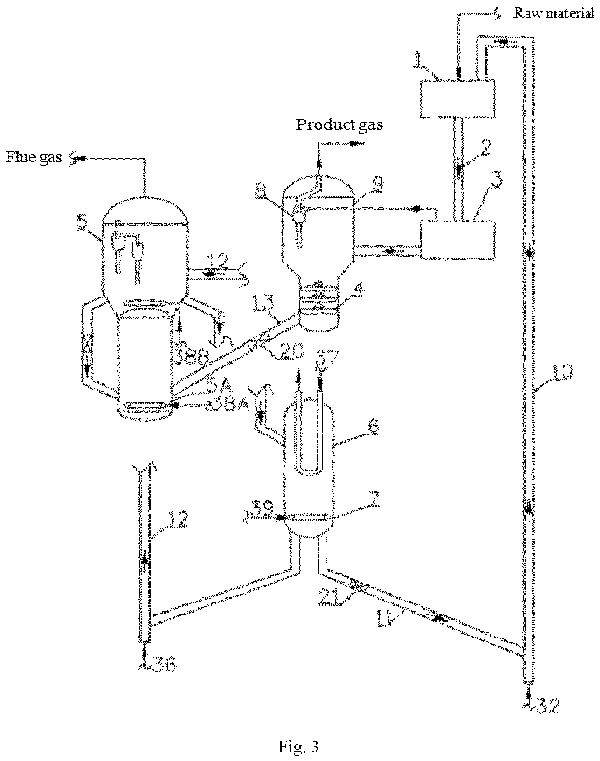

In order to achieve optimal control of the reaction temperature and optimal control of the reaction depth, the catalyst mixing and buffering space 7 is arranged at the downstream location of the regenerated catalyst cooler 6 to enhance the mixing of the regenerated catalyst so that the regenerated catalyst reaches temperature equilibrium before entering the downer reactor 2, so as to meet requirements of downstream reaction temperature control. In order to save space and investment, the catalyst mixing and buffering space 7 may also be an integrated structure with the regenerated catalyst cooler 6 and having a same diameter as that of the regenerated catalyst cooler 6 (as shown in Fig. 3). The catalyst mixing and buffering space 7 is operated by a low-velocity dense-phase fluidized bed having a superficial gas velocity of less than 0.3 m/s (preferably 0.0001-0.1999 m/s).

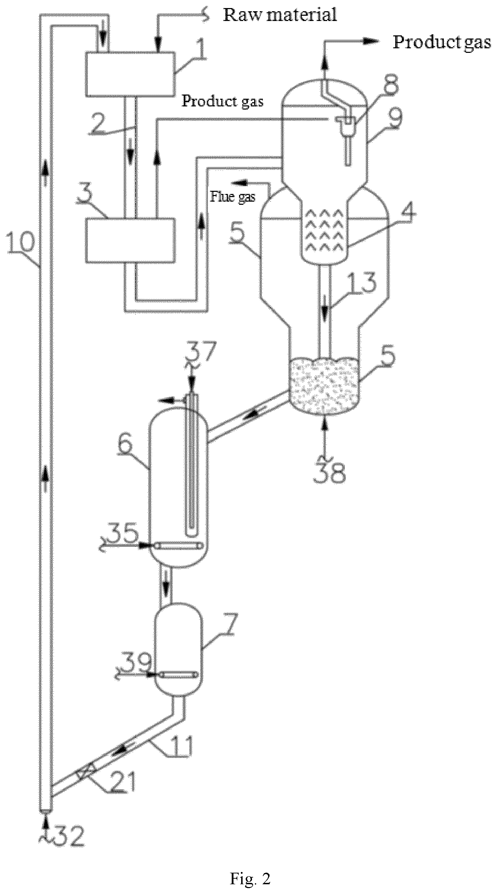

Fig. 2 is a schematic flow chart of a device for catalytic conversion using a downer reactor in a coaxial regeneration mode according to the present invention.

As shown in Fig. 2, the method for catalytic conversion and the device thereof according to the present invention comprise an inlet end 1 of a downer reactor, the downer reactor 2, a rapid separation unit 3, a spent catalyst stripper 4, a regenerator 5, a regenerated catalyst cooler 6, a catalyst mixing and buffering space 7, a secondary separator 8, and a settler 9.

The regenerator 5 is connected to the regenerated catalyst cooler 6 through a regenerated catalyst conveying pipe. The regenerated catalyst, after being cooled by the regenerated catalyst cooler 6 and then mixed and buffered in the catalyst mixing and buffering space 7 located at the downstream location (or at a lower portion), is connected to the inlet end 1 of the downer reactor through a cold regenerated catalyst conveying pipe 10. The temperature of the cold regenerated catalyst leaving the regenerated catalyst cooler 6 is controlled by adjusting a flow rate of a fluidizing medium 35 (including air, a steam, etc.). A control valve 21 is a specific control element arranged to facilitate control of the flow rate of the cold regenerated catalyst. The conveying medium 35 may be a steam, or other gases, or a mixture thereof (preferably a steam). A heat-removing medium 37 used may be water, a steam, air or other gases, various oils, or a mixture thereof.

In order to facilitate control of the temperature of the regenerated catalyst entering the downer reactor, it is also possible to provide a hot regenerated catalyst bypass pipe (including a control valve) (not shown in the figure) directly connected to the catalyst mixing and buffering space 7 in which the cooled regenerated catalyst and the hot regenerated catalyst are thoroughly mixed to reach temperature equilibrium.

There are of course many other control devices and control methods, and the implementation of the inventive concept of the present invention is not limited in this respect.

A hydrocarbon raw material and the regenerated catalyst, after being mixed at the inlet end 1 of the reactor, enters the downer reactor 2, and go into a reaction under catalytic conversion conditions. The main operating conditions are as follows: a reaction temperature of 460-680°C (preferably 480-660°C, most preferably 490-630°C); a reaction pressure of 0.11-0.4 MPa; a contact time of 0.05-2 seconds (preferably 0.1-1.5 seconds); and a weight ratio of the catalyst to the raw material (catalyst-to-oil ratio) of 6-50 (preferably 8-40).

When the reaction oil and gas and the catalyst flow, in a mixed manner, co-currently downward to the rapid separation unit 3 at the tail end of the downer reactor 2, the rapid separation unit 3 rapidly separates the catalyst and the product oil or gas; or the high-temperature product oil and gas is quenched (to avoid a secondary reaction; not shown in the figure) and enters the secondary separator 8 (such as a cyclone separator) for further removal of the catalyst, and then enters a downstream fractionation or separation system for further separation, to obtain desired gas products and liquid products.

The spent catalyst, after being passed through the settler 9 and stripped by the spent catalyst stripper 4, enters the regenerator 5 through a spent catalyst conveying pipe 13 and a control valve (not shown in the figure), and is burned in the presence of a main wind 38 (an oxygen-containing gas, including such as air etc.). The regenerated catalyst is led out from a lower portion of the regenerator 5, enters the regenerated catalyst cooler 6, and then enters the catalyst mixing and buffering space 7 for thorough mixing. After that, the cold regenerated catalyst is recycled for reuse by way of a conveying pipe 11 (or mixed with the hot regenerated catalyst) (it is also possible to provide another catalyst conveying pipe to return the catalyst to the regenerator). (Of course, a separate external heat exchanger may also be arranged depending on process requirements to allow flexible operations under multiple operating conditions).

The regenerated catalyst from the regenerator 5 enters the regenerated catalyst cooler 6 and is cooled to 200-720°C. The cold regenerated catalyst leaving the lower portion or the bottom of the regenerated catalyst cooler 6 is mixed and buffered in the catalyst mixing and buffering space 7 to reach temperature equilibrium, and then returned and recycled to the inlet end 1 of the downer reactor and the reactor 2 for reuse. The fluidizing medium 39 may be air, a steam, or other gases, or a mixture thereof (preferably a steam).

In order to achieve precise control and optimal control of the reaction temperature, the catalyst mixing and buffering space 7 is arranged at the downstream location of the regenerated catalyst cooler 6 to enhance the mixing of the regenerated catalyst, so that the regenerated catalyst reaches temperature equilibrium before entering the inlet end 1 of the downer reactor and the reactor 2, so as to meet the requirements of precise downstream reaction temperature control. In order to save space and investment, the catalyst mixing and buffering space 7 and the regenerated catalyst cooler 6 may also be designed as an integrated structure, in which 6 the catalyst mixing and buffering space 7 has a same diameter as that of the regenerated catalyst cooler 6. The catalyst mixing and buffering space 7 is operated by a low-velocity dense-phase fluidized bed having a superficial gas velocity of less than 0.3 m/s (preferably 0.0001 to 0.1999 m/s).

Fig. 3 is a schematic flow chart of a device for catalytic conversion using a downer reactor in a fast bed regeneration mode according to the present invention.

As shown in Fig. 3, the method for catalytic conversion and the device thereof according to the present invention comprise an inlet end 1 of the downer reactor, the downer reactor 2, a rapid separation unit 3, a spent catalyst stripper 4, a regenerator 5, a regenerated catalyst cooler 6, a catalyst mixing and buffering space 7, a secondary separator 8, and a settler 9.

The regenerator 5 is connected to the regenerated catalyst cooler 6 through a regenerated catalyst conveying pipe. The regenerated catalyst, after being cooled by the regenerated catalyst cooler 6 and mixed and buffered in the catalyst mixing and buffering space 7 located at the downstream location (or at a lower portion) to reach temperature equilibrium, is connected to the inlet end 1 of the downer reactor through a cold regenerated catalyst conveying pipe 10. The temperature of the cold regenerated catalyst leaving the regenerated catalyst cooler 6 is controlled by adjusting the flow rate of a fluidized medium 35 (including air, a steam, etc.). A control valve 21 is a specific control element arranged to facilitate control of the flow rate of the cold regenerated catalyst.

In order to facilitate control of the temperature of the regenerated catalyst entering the downer reactor, it is also possible to provide a hot regenerated catalyst conveying pipe (including a control valve and not shown) directly connected to the regenerated catalyst mixing and buffering space 7 in which the cold regenerated catalyst and the hot regenerated catalyst are mixed to reach temperature equilibrium.

There are of course many other control devices and control methods, and the implementation of the inventive concept of the present invention is not limited in this respect.

The catalyst cooler described above may be integrated with the regenerator or the downer reactor or may be connected thereto through pipelines.

The hydrocarbon raw material and the regenerated catalyst, after being mixed, enter the downer reactor, and go into a reaction under catalytic conversion conditions. The main operating conditions are as follows: a reaction temperature of 460-680°C (preferably 480-660°C, most preferably 490-630°C); a reaction pressure of 0.11-0.4 MPa; a contact time of 0.05-2 seconds (preferably 0.1-1.5 seconds); and a weight ratio of the catalyst to the raw material (the catalyst-to-oil ratio) of 6-50 (preferably 8-40).

The reaction oil and gas and the catalyst flow, in a mixed manner, co-currently down to the rapid separation unit 3 at the tail end of the downer reactor 2. The high temperature oil and gas from the rapid separation unit 3, then enters or after being quenched enters (to avoid a secondary reaction, not shown in the figure), the secondary separator 8 (such as a cyclone separator) for further removal of the catalyst, then a high temperature oil and gas from the secondary separator 8 enters a downstream fractionation or separation system for further separation, or is quenched again (to avoid a secondary reaction, not shown in the figure) and then enters a downstream fractionation or separation system for further separation, to obtain desired gas products and liquid products.

The separated spent catalyst, after being stripped by the spent catalyst stripper 4, enters a coke burning tank 5A through a spent catalyst conveying pipe 13 and a control valve 20, and is rapidly burned in the presence of a main wind 38A (an oxygen-containing gas, including such as air etc.), and is then sent upward to the regenerator 5 and is further burned for regeneration. The bottom of the regenerator 5 is supplemented with a secondary wind 38B (an oxygen-containing gas, including such as air etc.). The regenerated catalyst is led out from the lower portion of the regenerator 5, and enters the regenerated catalyst cooler 6 and the regenerated catalyst mixing and buffering space 7. The cold regenerated catalyst is recycled for reuse by way of the conveying pipe 11 (or mixed with the hot regenerated catalyst). Another conveying pipe 12 may be arranged (the conveying pipe 12 may not be arranged ) to return the catalyst to the regenerator (Of course, a separate external heat exchanger may be arranged depending on process requirements to allow flexible operations under multiple operating conditions).

The regenerated catalyst from the regenerator 5 enters the regenerated catalyst cooler 6 and is cooled to 200-720°C. The cold regenerated catalyst leaving the lower portion or the bottom of the regenerated catalyst cooler 6 is mixed and buffered in the catalyst mixing and buffering space 7 to reach temperature equilibrium, and then returned and recycled to the inlet end of the downer reactor and the reactor 2 for reuse. The fluidizing medium 39 may be air, a steam, or other gases, or a mixture thereof (preferably a steam). The heat-removing medium 37 may be water, a steam, air or other gases, various oils, or a mixture thereof. The conveying medium 36 may be air, a steam, or other gases, or a mixture thereof.

In order to achieve precise control and optimal control of the reaction temperature, the mixing buffer space 7 is arranged at the downstream location of the regenerated catalyst cooler to enhance the mixing of the regenerated catalyst, so that the regenerated catalyst reaches temperature equilibrium before entering the downer reactor 2, to meet requirements of downstream reaction temperature control. In order to save space and investment, the catalyst mixing and buffering space 7 and the regenerated catalyst cooler 6 may also be designed as an integrated structure, in which the catalyst mixing and buffering space 7 has a same diameter as that of the regenerated catalyst cooler 6. The catalyst mixing and buffering space 7 is operated by a low-velocity dense-phase fluidized bed having a superficial gas velocity of less than 0.3 m/s (preferably 0.0001-0.1999 m/s).

### Detailed Description of the Embodiments

Before a further detailed description of the specific embodiments of the present invention, it should be appreciated that the scope of the present invention is not limited to the specific embodiments described below, and that the terms in the embodiments of the present invention are used for the purpose of describing particular embodiments only and are not intended to limit the scope of the present invention.

It should be appreciated that that, unless otherwise indicated by the present invention, when numerical ranges are given in the embodiments, both endpoints of each numerical range and any numerical value between the two endpoints are optional. Unless otherwise defined, all technical and scientific terms used in the present invention have the same meaning as commonly understood by those skilled in the art. The present invention may be implemented using any existing methods, devices, or materials similar to or equivalent to methods, devices, and materials used in the embodiments of the present invention based on the knowledge of those skilled in the art about the existing technologies and the disclosure of the present invention, in addition to the specific methods, devices, and materials used in the embodiments.

### Example 1

In order to verify the effects of the present invention, the process flow shown in Fig. 2 or Fig. 3, a heavy oil raw material having properties shown in Table 1, process conditions of the existing technology and the present invention shown in Table 2, as well as a molecular sieve catalyst (CHP-1) that maximizes propylene production were used. Test results are listed in Table 3.

The results in Table 3 show that, due to the thermal equilibrium limitation of the existing downer reactor, the existing technology can achieve a catalyst-to-oil ratio of only up to 11.5 at a feed temperature of 230°C, and thus cannot realize the large catalyst-to-oil ratio operation required by a downer reactor. Compared with the existing technology, the present invention decreases the yield of coke and dry gas by 1.8 percentage points, increases the total yield of light oil (LPG + gasoline + diesel) by 2.1 percentage points, increases the total yield of liquids (total yield of light oil + oil slurry) by 1.8 percentage points, and also decreases the yield of diesel by 10 percentage points, and thus realizes a good product distribution. For a set of 1 million tons/year heavy oil catalytic cracking unit, the present invention can bring an annual economic increase of about 90 million RMB.

**Table 1 Properties of heavy oil raw material**

| Heavy oil raw material | |
|---|---|
| Density, 20/4°C | 0.881 |
| Residual carbon | 0.4% (Wt) |
| Sulfur content | 0.15% (Wt) |

**Table 2 Comparison of process conditions of the existing technology and the present invention**

| Parameters | Existing technology | Present invention |
|---|---|---|
| | Solution A | Solution B |
| reaction temperature, °C | 560 | 560 |
| feed temperature, °C | 230 | 280 |
| regeneration temperature, °C | 700 | 700 |
| temperature of regenerated catalyst entering the downer reactor, °C | 695 | 648 |
| catalyst-to-oil ratio, weight/weight | 11.5 | 16.1 |
| reaction time, second | 1.1 | 0.8 |

**Table 3**

| Products | Existing technology | Present invention |
|---|---|---|
| product yield Wt% | | |
| fuel gas + H₂S | 5.1 | 4.1 |
| LPG | 13.1 | 25.1 |
| gasoline | 38.5 | 39.2 |
| diesel | 29.7 | 19.1 |
| oil slurry | 5.3 | 5.0 |
| coke | 8.3 | 7.5 |
| Total | 100.0 | 100.0 |
| Total yield of light oil | 81.3 | 83.4 |
| Total yield of liquids | 86.6 | 88.4 |

### Example 2

In Example 2, the raw material for the downer reactor was catalytic cracking gasoline, and the downer reactor needed to adopt an ultra-large catalyst-to-oil ratio of about 30 to optimize the reaction temperature distribution, improve the reaction selectivity, increase the yields of propylene and gasoline, and improve the aromatic content in gasoline and the octane number of gasoline, and reduce the olefins content in gasoline.

This example adopts the process flow shown in Fig. 2 or Fig. 3, process conditions of the existing technology and the present invention shown in Table 4, as well as a molecular sieve catalyst (CHP-1) that maximizes propylene production. The gasoline raw material and test results are listed in Table 5.

The results in Table 4 indicate that, due to the thermal equilibrium limitation of the existing downer reactor, solution B of the existing technology realizes a catalyst-to-oil ratio of only 12.1 at a feed temperature of 400°C under thermal equilibrium. Such a catalyst-to-oil ratio will severely affect the conversion rate and selectivity of the downer reactor. Although solution A can increase the catalyst-to-oil ratio to 25.9 at a feed temperature of 40°C, it will seriously affect the recovery and utilization of the low temperature heat in the device.

The results in Table 5 show that, compared with the existing technology, the present invention increases the yield of high value-added propylene by 1.2 percentage points, realizes quite a similar yield of coke and dry gas and quite a similar total yield of light oil, decreases the aromatics content in gasoline by 3.7 percentage points, reduces the olefins content by 3 percentage points, and increases the octane number (RON) by 0.8-2 units. Further, Table 4 shows that the present invention can also maximize the recovery efficiency of waste heat due to the use of a high-temperature feed at 400°C.

All these indicate that the method for catalytic conversion of gasoline according to the present invention produces significant beneficial effects.

**Table 4 Properties of gasoline raw material**

| Parameters | Existing technology | | Present invention |
|---|---|---|---|
| | Solution A | Solution B | |
| reaction temperature, °C | 610 | 610 | 610 |
| feed temperature, °C | 400 | 400 | 400 |
| regeneration temperature, °C | 690 | 690 | 690 |
| temperature of regenerated catalyst entering the downer reactor, °C | 685 | 685 | 652 |
| Catalyst-to-oil ratio, weight/weight | 25.9 | 12.1 | 30 |
| reaction time, second | 0.6 | 0.6 | 0.6 |

**Table 5 Comparison of process conditions of the existing technology and the present invention**

| Products | Properties of raw material | Solution A of existing technology | Solution B of existing technology |
|---|---|---|---|
| product yield Wt% | | | |
| dry gas | | 3.6 | 3.7 |
| LPG | | 28.6 | 31.5 |
| *wherein propylene* | | *10.1* | *11.3* |
| gasoline | | 64.3 | 61.5 |
| diesel | | | |
| coke | | 3.5 | 3.3 |
| Total | | 100.0 | 100.0 |
| Total yield of light oil (including LPG) | | 92.9 | 93 |
| Properties of gasoline | | | |
| Group composition: (V%) | | | |
| olefins | 43.6 | 15.8 | 12.8 |
| aromatic hydrocarbons | 8.1 | 39.6 | 43.3 |
| alkanes | 34.8 | 31.7 | 31.1 |
| cycloalkanes | 8.2 | 8.0 | 7.9 |
| others | 5.3 | 4.9 | 4.9 |
| Total | 100 | 100 | 100 |
| octane number (RON) | 88.3 | 90.3 | 91.1 |

### Example 3

In Example 3, the raw material for the downer reactor was an olefin-rich mixed C4. This example adopted the process flow shown in Fig. 2 or Fig. 3, and the process conditions of the existing technology and the present invention shown in Table 6, as well as a molecular sieve catalyst ZSM-5.

A downer reactor usually needs to adopt an ultra-large catalyst-to-oil ratio of over 30 to achieve the purposes of optimizing the reaction temperature distribution, improving the reaction selectivity, and increasing the yield of ethylene and propylene. However, the results in Table 6 indicate that due to the thermal equilibrium limitation of the existing downer reactor, the existing technology realizes a catalyst-to-oil ratio of only 16.3 at a high feed temperature of 400°C under thermal equilibrium. Such a low catalyst-to-oil ratio will severely affect the conversion rate and selectivity of the downer reactor.

The mixed C4 raw material used in the present invention and test results are shown in Table 7. Table 7 shows that the yield of high value-added propylene is about 47.2%, and the yield of ethylene is 10.6%, indicating that the method for catalytic conversion of the mixed C4 according to the present invention produces remarkable beneficial effects.

**Table 6 Comparison of process conditions of the existing technology and the present invention**

| Parameters | Existing technology | Present invention |
|---|---|---|
| reaction temperature, °C | 620 | 620 |
| feed temperature, °C | 400 | 400 |
| regeneration temperature, °C | 700 | 700 |
| temperature of regenerated catalyst entering the downer reactor, °C | 695 | 655 |
| catalyst-to-oil ratio, weight/weight | 16.5 | 30 |
| reaction time, second | 0.6 | 0.6 |

**Table 7 Properties of mixed C4 raw material and test results**

| Items | Parameters | Present invention |
|---|---|---|
| Components of raw material: mol% | | |
| C₃+C₅ | 0.6 | |
| butane | 11.9 | |
| butene | 87.5 | |
| Total | 100 | |
| <C2 | | 15.8 |
| *wherein ethylene* | | 10.6 |
| propane | | 4.3 |
| propylene | | 47.2 |
| butane | | 10.3 |
| butene | | 11.7 |
| liquid + coke + loss | | 10.7 |
| *wherein ethylene* + *propylene* | | 57.8 |
| Total | | 100 |

The above embodiments are merely illustrative of the principles and advantages of the present invention, and are not to limit the present invention. Any skilled in the art can make modifications and changes to the embodiments described above without departing from the spirit and scope of the present invention. Accordingly, any equivalent modifications or changes made by those skilled in the art without departing from the spirit and technical concepts of the present invention should be covered by the appended claims.

## Claims

1. A method for catalytic conversion of a hydrocarbon using a downer reactor, wherein a regenerated catalyst from a regenerator, after being cooled by a regenerated catalyst cooler, enters a downer reactor and is mixed and contacted with a hydrocarbon raw material at an inlet end of the downer reactor; the regenerated catalyst and the hydrocarbon raw material go on a catalytic conversion reaction of the hydrocarbon in the downer reactor, and flow co-currently downward to a tail end of the downer reactor for rapid separation; a separated spent catalyst, after being stripped, enters a regenerator and is burned for regeneration to form a regenerated catalyst, and the regenerated catalyst, after being cooled by a regenerated catalyst cooler, is returned and recycled to the downer reactor for reuse.

2. The method according to claim 1, wherein the downer reactor is operated under the following main conditions: a reaction temperature of 460-680°C, a reaction pressure of 0.11-0.4 MPa, a contact time of 0.05-2 seconds, and a catalyst-to-oil ratio of 6-50.

3. The method according to claim 1, wherein a specific process of the method is as follows:
1) the hydrocarbon raw material, after being preheated or being not preheated, and the low-temperature regenerated catalyst from the regenerated catalyst cooler enter the inlet end of the downer reactor, and react while flowing downward along the reactor; when a mixture of a reacting oil and gas and the catalyst flows downward to the tail end of the reactor, a rapid separation is performed to realize rapid separation of the catalyst and the oil and gas, wherein the downer reactor is operated under the following main conditions: a reaction temperature of 460-680°C, a reaction pressure of 0.11-0.4 MPa, a contact time of 0.05-2 seconds, and a catalyst-to-oil ratio of 6-50;
2) the separated spent catalyst, after being stripped by a spent catalyst stripper, enters the regenerator and is burned for regeneration, wherein a regeneration temperature is controlled at 630-730°C;
3) the regenerated catalyst from the regenerator enters the regenerated catalyst cooler and is cooled to 200-720°C, and the cooled regenerated catalyst is recycled to the inlet end of the downer reactor for reuse; or a hot regenerated catalyst bypass is arranged, so that a portion of the hot regenerated catalyst is mixed with the cold regenerated catalyst and then a mixed regenerated catalyst is recycled to the inlet end of the downer reactor for reuse.

4. The method according to claim 2 or 3, wherein the downer reactor is operated under conditions of: a reaction temperature of 490-650°C, a reaction pressure of 0.11-0.4 MPa, a contact time of 0.1-1.5 seconds, and a catalyst-to-oil ratio of 8-40.

5. The method according to any one of claims 1 to 4, wherein optimization of the reaction temperature of the downer reactor is achieved by adjusting a temperature of the regenerated catalyst entering the downer reactor.

6. The method according to any one of claims 1 to 5, wherein a catalyst mixing and buffering space is arranged at the downstream location of the regenerated catalyst cooler, wherein the catalyst mixing and buffering space is operated by a low-velocity dense-phase fluidized bed having a superficial gas velocity of less than 0.3 m/s.

7. The method according to any one of claims 1 to 6, wherein a reaction temperature of the downer reactor is controlled by adjusting a catalyst-to-oil ratio, or/and by adjusting a temperature of a cold regenerated catalyst or a temperature of a mixed regenerated catalyst.

8. The method according to claim 5 or 7, wherein the temperature of the mixed regenerated catalyst is independently controlled by adjusting proportions of the cold regenerated catalyst and the hot regenerated catalyst; or the temperature of the cold regenerated catalyst is controlled by adjusting a flow rate of a fluidizing medium and/or of a heat-removing medium, or by adjusting a flow rate of a fluidizing medium and/or of a heat-removing medium and/or of the cold catalyst returned to the regenerator.

9. The method according to any one of claims 1 to 8, wherein the hydrocarbon raw material is any heavy oil having been hydrogenated or having not been hydrogenated, including one of straight-run gas oil, coking gas oil, hydrocracked tail oil, atmospheric pressure residual oil, vacuum residual oil, shale oil, synthetic oil, crude oil, coal tar, recycle oil, oil slurry, deasphalted oil, thermal cracking heavy oil, viscosity-reduced heavy oil, heavy diesel, and the like, or a mixture of two or more than two thereof; the straight-run gas oil fraction or the coking gas oil fraction includes high-density cycloalkyl or naphthenic intermediate gas oil (distillate oil), and is a full-range fraction or a partial narrow fraction thereof; or the hydrocarbon raw material is a light hydrocarbon raw material, which is an olefin-containing hydrocarbon or saturated liquid light hydrocarbon in a refinery or a petrochemical plant, including any one of liquefied petroleum gas, light oil, and the like, or a mixture of more than one thereof in any ratio; the liquid light hydrocarbon is C4 and C5 fractions containing butene and pentene, or a mixture thereof in any ratio; the light oil is a gasoline fraction, including one or two or more than two of straight-run gasoline, gas condensate, catalytic cracking gasoline, thermal cracking gasoline, viscosity-reduced gasoline, coking gasoline, pyrolysis gasoline, or a mixture gasoline thereof in any ratio, and is a full-range gasoline or a partial narrow fraction thereof; or the light oil is a diesel fraction, including catalytic cracking diesel, and is a full-range diesel or a partial narrow fraction thereof.

10. The method according to any one of claims 1 to 9, wherein the method is implemented separately, or the downer reactor is coupled to a riser reactor, wherein a gas-solid co-currently flowing folding-type fast fluidized bed reactor or a gas-solid co-current down-flow and up-flow coupled catalytic cracking reactor is used.

11. A device for catalytic conversion of a hydrocarbon using a downer reactor, wherein the device includes the downer reactor, a rapid separation unit, a spent catalyst stripper, a regenerator, a regenerated catalyst cooler, wherein the downer reactor is a gas-solid co-currently flowing folding-type fast fluidized bed reactor or a gas-solid co-current down-flow and up-flow coupled catalytic cracking reactor.

12. The device according to claim 11, wherein a catalyst mixing and buffering space is arranged at the downstream location of the regenerated catalyst cooler.
